(19) Europäisches Patentamt

European Patent Office

Office européen des brevets

(11) **EP 0 801 052 B1**

(12) **EUROPÄISCHE PATENTSCHRIFT**

(45) Veröffentlichungstag und Bekanntmachung des Hinweises auf die Patenterteilung:
**16.02.2000 Patentblatt 2000/07**

(51) Int Cl.$^7$: **C07C 68/08**, C07C 68/00, C07C 69/96

(21) Anmeldenummer: **97105175.0**

(22) Anmeldetag: **27.03.1997**

(54) **Rückgewinnung von Katalysatorsystemen aus diarylcarbonathaltigen Reaktionsgemischen durch Schmelzkristallisation**

Recovery of catalyst components from diarylcarbonate containing reaction mixtures by melt crystallization

Récupération de compositions catalytiques de mélanges réactionnels contenant du carbonate de diaryle par cristallisation d'un mélange en fusion

(84) Benannte Vertragsstaaten:
**BE DE ES FR GB IT NL**

(30) Priorität: **09.04.1996 DE 19613973**

(43) Veröffentlichungstag der Anmeldung:
**15.10.1997 Patentblatt 1997/42**

(73) Patentinhaber: **BAYER AG**
**51368 Leverkusen (DE)**

(72) Erfinder:
• **Buysch, Hans-Josef, Dr.**
 **47809 Krefeld (DE)**
• **Hesse, Carsten, Dr.**
 **47800 Krefeld (DE)**
• **Rechner, Johann, Dr.**
 **47906 Kempen (DE)**
• **Wirges, Hans-Peter, Dr.**
 **47800 Krefeld (DE)**

(56) Entgegenhaltungen:
**EP-A- 0 687 666**      **US-A- 5 239 106**

**Beschreibung**

Die vorliegende Erfindung betrifft ein Verfahren zur Rückgewinnung von Katalysatorsystemen aus diarylcarbonathaltigen Reaktionsgemischen durch Schmelzkristallisation, wobei man eine das Katalysatorsystem enthaltende Restschmelze und ein Schmelzkristallisat erhält. Die Restschmelze kann in den Reaktor zur Herstellung des Diarylcarbonats zurückgeführt oder auf Wertstoffe aufgearbeitet werden. Das Schmelzkristallisat wird auf reines Diarylcarbonat und reine Hydroxyverbindung aufgearbeitet.

US-PS 5 239 106 nennt die Abtrennung von Diphenylcarbonat aus katalysatorhaltigen Reaktionslösungen durch Kristallisation des 1:1 Addukts mit Phenol, bestehend aus 30,5 Gew.-% Phenol und 69,5 Gew.-% Diphenylcarbonat, aus Reaktionsgemischen mit Hilfe der Suspensionskristallisation, Nachteilig bei diesem Verfahren ist die Beschränkung auf einen engen Konzentrationsbereich, um das 1:1-Addukt mit ausreichend hoher Ausbeute isolieren zu können, d.h. Diphenylcarbonatkonzentrationen von mindestens 50 Gew.-% bis 70 Gew.-%. Um die resultierenden Suspensionen noch filtrationstechnisch verarbeiten zu können, ist mindestens eine Zweistufigkeit des Verfahrens mit einem erheblichen apparativen Aufwand notwendig. Weiterhin läßt sich das Katalysatorsystem in diesem Verfahren nicht vollständig abtrennen, da die abfiltrierten Kristalle noch anhaftende Mutterlauge und Einschlüsse von Mutterlauge besitzen. Bei der anschließenden destillativen Aufarbeitung des 1:1-Addukts wirken sich diese nicht abgetrennten Katalysatorbestandteile durch die Katalyse von Nebenproduktbildung und der DPC-Zersetzung negativ aus. Die vorgeschlagene Waschung des Kristallisats mit einem Gemisch aus 9 % Wasser und 91 % Phenol reduziert die Ausbeute durch Lösen großer Teile des 1:1-Addukts. Weiterhin führt diese Behandlung zu einer Erhöhung des Wassergehalts der Adduktkristalle, was in den nachgeschalteten Destillationskolonnen, d.h. zur DPC-Isolierung und zur Wasserabtrennung aus der benutzten Waschlösung, zu DPC-Verlusten durch Hydrolyse führt. Weiterhin ist die Beschreibung des Verfahrens von US-PS 5 239 106 nicht ohne eigenes Zutun nachvollziehbar, da wesentliche Parameter dort nicht genannt sind. So fehlen Angaben zur Impftechnik, Art und Menge des Impfgutes, zum Typ des Kristallisators, etwa Rührkessel, Umlaufkristallisator und andere, zur Rührtechnik, wie Rührertyp, Rührgeometrie, spezifische Rührleistung, zur Kühltechnik, wie Kühlrate, Starttemperatur und Endtemperatur des Kühlens sowie zu Haltezeiten und Nachrührzeiten, etwa bei der Kühlendtemperatur.

Um auch Reaktionslösungen mit einem DPC-Gehalt von weniger als 50 Gew.-% verarbeiten zu können, ist eine destillative Anreicherung mit den oben geschilderten Nachteilen einer Destillation in Anwesenheit von Katalysatorbestandteilen unerläßlich. Zusätzlich führt eine thermische Belastung der Reaktionslösung zu einer Desaktivierung des Katalysatorsystems, was eine teure Frischeinspeisung der Katalysatorkomponenten in den Prozeß erfordert. Alle diese geschilderten Nachteile machen das Verfahren unflexibel, unattraktiv und stehen einer technischen Realisierung im Weg.

In EP-A 687 666 wird ein Verfahren zur Reinigung von Diphenylcarbonat durch fraktionierte Schmelzkristallisation von hochkonzentrierten Reaktionsgemischen im Temperaturbereich von 45-85°C beschrieben. Die erzielbaren Diphenylcarbonatreinheiten liegen zwischen 97,5 und 99,5 %. Nachteilig an diesem Verfahren ist die Beschränkung auf Reaktionsgemische mit einem Diarylcarbonatgehalt von mehr als 70 Gew.-%. Reaktionslösungen mit Diarylcarbonatgehalten unter 70 Gew.-% lassen sich nach diesem Verfahren nicht verarbeiten und müßten daher beispielsweise durch Destillation auf die erforderlichen Gehalte aufkonzentriert werden. Bei dieser thermischen Belastung verursacht das Katalysatorsystem Nebenreaktionen und wird dabei selbst desaktiviert. Das Verfahren ist aus diesen Gründen für Reaktionslösungen mit Diphenylcarbonatgehalten unter 70 Gew.-% unwirtschaftlich und umständlich.

Es bestand daher die Aufgabe, eine schonende Methode zu finden, die es erlaubt, Katalysatorsysteme aus diarylcarbonathaltigen Reaktionslösungen mit unterschiedlichen Diarylcarbonatgehalten mit hoher Raum-Zeit-Ausbeute ohne Desaktivierung des Katalysatorsystems und unter wirtschaftlichen, technisch realisierbaren und reproduzierbaren Bedingungen abzutrennen und zurückzugewinnen.

Es wurde nun gefunden, daß die beschriebenen Nachteile überwunden werden können, wenn man das Reaktionsgemisch aus dem Reaktor entnimmt, in einer fraktionierten Schmelzkristallisation durch Temperaturabsenkung und Impfen der Reaktionslösung eine katalysatorhaltige Schmelze gewinnt, Reste des Katalysatorsystems aus dem Kristallisat durch Schwitzen abtrennt, die so vom Katalysatorsystem und anderen Verunreinigungen befreiten Kristallisate, bestehend aus einem Gemisch aus Diarylcarbonat und aromatischer Hydroxyverbindung, verlustfrei durch Kristallisation oder Destillation in hochreines Diarylcarbonat aufarbeitet und die das Katalysatorsystem enthaltende Reaktionslösung in den Reaktor zurückführt. Überraschenderweise wurde im Falle des Diarylcarbonat/Phenol-Systems gefunden, das sich die Zusammensetzung der Kristallisate, in Abhängigkeit vom Diphenylcarbonatgehalt der Reaktionslösung, ändert und nur in einem sehr engen Konzentrationsbereich das 1:1-Addukt auftrat. Eine Waschung der erhaltenen Kristallisate ist nicht erforderlich. Weiterhin findet keine thermische Schädigung des Katalysatorsystems statt, was die Katalysatordesaktivierung auf ein Minimum reduziert. Ein besonderer Vorteil des erfindungsgemäßen Verfahrens besteht darin, daß es einstufig ohne einen Filtrationsschritt durchführbar ist. Weiterhin können Reaktionslösungen, deren Diarylcarbonatgehalt in einem weiten Bereich von 15 bis 70 % liegt, eingesetzt werden. Außerdem können verschiedene Diarylcarbonate in den einsetzbaren Reaktionslösungen enthalten sein.

Die Erfindung betrifft demnach eine Methode zur Rückgewinnung von Katalysatorsystemen, die einen Platingruppenmetall-Katalysator, einen Cokatalysator, ein quaternäres Salz und eine Base enthalten, aus Reaktionsgemischen zur Herstellung von Diarylcarbonaten der Formel (I)

$$R\text{-}O\text{-}CO\text{-}O\text{-}R \hspace{4cm} (I)$$

durch oxidative Carbonylierung der zugrundeliegenden aromatischen Hydroxyverbindungen der Formel (II)

$$R\text{-}O\text{-}H \hspace{5cm} (II),$$

wobei in den Formeln

R   substituiertes oder nichtsubstituiertes $C_6$-$C_{15}$-Aryl, bevorzugt substituiertes oder nichtsubstituiertes Phenyl, besonders bevorzugt nichtsubstituiertes Phenyl bedeutet,

und die einen Diarylcarbonatgehalt von mindestens 15 und weniger als 70 Gew.-%, bezogen auf das Gesamtgewicht der Reaktionsgemische, aufweist,

die dadurch gekennzeichnet ist, daß man

a) das Reaktionsgemisch aus dem Reaktor zur Herstellung des Diarylcarbonats in einen zur Schmelzkristallisation geeigneten Apparat überführt,

b) im geeigneten Apparat durch Temperaturabsenkung und Impfen die Schmelzkristallisation einleitet,

c) das Schmelzkristallisat, das überwiegend aus Diarylcarbonat und der zugrundeliegenden aromatischen Hydroxyverbindung besteht, von der zurückbleibenden katalysatorhaltigen Restschmelze abtrennt,

d) das Schmelzkristallisat auf reines Diarylcarbonat und reine aromatische Hydroxyverbindung aufarbeitet und

e) die katalysatorhaltige Restschmelze in den Reaktor zur Herstellung des Diarylcarbonats zurückführt oder zur Gewinnung von Wertstoffen aufarbeitet.

R ist $C_6$-$C_{15}$-Aryl, wie Phenyl, Biphenylyl, Naphthyl, Anthryl oder HO-$C_6H_4$-HO-$C_6H_4$-C$(CH_3)_2$-$C_6H_4$- (Rest von Bishenol A), bevorzugt Phenyl. Die aromatischen Kerne können jeweils ein- oder zweimal durch -$CH_3$, -$C_2H_5$, -Cl, -Br oder -F substituiert sein; besonders bevorzugtes R ist nicht substituiertes Phenyl.
Zur Durchführung des erfindungsgemäßen Verfahrens können z.B. Rohrbündel-Kristallisatoren oder modifizierte Plattenwärmeaustauscher unterschiedlicher Konstruktion mit oder ohne Aufteilung der Schmelze, mit und ohne Anwendung von Pulsationen bzw. mit und ohne Unterteilung der Rohre in Segmente mit separater Ausspeisung verwendet werden. Weiterhin können Fallfilmkristallisatoren unterschiedlicher Bauweise, z.B. die aus der EP-A 218 545 bekannten, eingesetzt werden. Weitere einsetzbare Apparate sind Blasensäulenkristallisatoren, Kristallisierwalzen und -bänder. Alle Apparate haben Wärmeaustauschflächen und einen Kühlmittelkreislauf; die unten angegebenen Temperaturen sind die des von den Wärmeaustauschflächen zurücklaufenden Kühlmittels. Weitere Einzelheiten einer kontinuierlich arbeitenden, geeigneten Kristallisationseinrichtung finden sich in der EP-A 521 499.
Bevorzugt werden für das erfindungsgemäße Verfahren Rohrbündel-Kristallisatoren, Plattenwärmeaustauscher unterschiedlicher Konstruktion mit und ohne Umpumpen der Schmelze oder Fallfilmkristallisatoren eingesetzt.
Der Kristallisationsvorgang im erfindungsgemäßen Verfahren wird durch eine definierte Zufuhr von Kristallisationskeimen (Impfung) eingeleitet.
Das erfindungsgemäße Verfahren kann mit destillativen Reinigungsverfahren verbunden werden. So ist es möglich, nach Anwendung der Schmelzkristallisation, eine Destillation von Diphenylcarbonat durchzuführen. Man kann das erfindungsgemäße Verfahren auch mit anderen einfachen Kristallisationsverfahren kombinieren, beispielsweise dem in der US-PS 5 239 106 beschriebenen.
Die folgenden Darstellungen sind beispielhaft auf Diphenylcarbonat (DPC) bezogen; es ist für den Fachmann jedoch einfach, die einzustellenden Bedingungen den physikalischen Daten anderer Diarylcarbonate anzupassen.
Im Fall der Ausführung des erfindungsgemäßen Verfahrens in Rohrbündel-Kristallisatoren oder Plattenwärmeaustauschern wird die Schmelze im Bereich von 53 bis 29°C mit einer Kühlrate von 20 bis 0,1K/h, bevorzugt 10 bis 0,5K/h abgekühlt. Während dieser Abkühlphase wird der Kristallisationsvorgang durch definierte Keimzufuhr (Impfung) bei einer Impftemperatur von 53 bis 29°C eingeleitet. Die Impfgutmasse beträgt 0,02 bis 1 % (bezogen auf eingesetztes Diphenylcarbonat). Die Kühlendtemperatur beträgt 20 - 48°C, bei dieser Temperatur wird gegebenenfalls vor der Abtrennung der Restschmelze eine Haltezeit von bis zu 100 Minuten eingehalten. Die Haltezeit beträgt daher unter Berücksichtigung beider Varianten 1 bis 5 Stunden. Danach erfolgt die Abtrennung der Restschmelze, und das Kristallisat wird durch Aufheizen mit einer Heizrate von 20 bis 0,1K/h, bevorzugt 10 bis 0,5K/h, bis zu einer Endtemperatur von 29 bis 75°C weiter gereinigt.

Zur Verbesserung der Reinigungsleistung können während dieser Aufheizphase gegebenenfalls weitere Haltepunkte zur Abtrennung weiterer Restschmelzeanteile dienen und bis zu diesen Haltepunkten abgeschmolzene Verunreinigungen zusammen mit der Restschmelze entfernt werden. In einer weiteren Ausführungsform kann während des Aufheizvorgangs, ohne Unterbrechung der Aufheizung, die zunächst anfallende Restschmelze mit den ausgeschwitzten restlichen Verunreinigungen abgetrennt und somit von der bei höherer Temperatur durch Aufschmelzen des Kristallisats anfallenden DPC-Reinschmelze getrennt werden.

Die Reinschmelze wird auf reines Diarylcarbonat und reine aromatische Hydroxyverbindung aufgearbeitet; dies kann beispielsweise durch Destillation, Lösungskristallisation, Extraktion oder andere bekannte Methoden geschehen. Die abgetrennte (Rest)Schmelze wird gemeinsam mit beim "Schwitzen" erhaltenen weiteren Teilschmelzen als Katalysatorsystem in den Reaktor zur Herstellung des Diarylcarbonats zurückgeführt oder zur Gewinnung von Wertstoffen, etwa des Platingruppenmetalls, aufgearbeitet. Die Zurückführung ist die wichtigere Verwendung.

Die nachfolgenden Beispiele sollen das erfindungsgemäße Vorgehen verdeutlichen, ohne es in irgendeiner Weise einzuschränken. Die Versuche zur fraktionierenden Schmelzkristallisation wurden in einer statisch betriebenen Säule (Durchmesser: 30 mm; Höhe: 900 mm) als Schichtkristallisation durchgeführt. Als Hauptverunreinigung wird in den Beispielen Tetrabutylammoniumbromid (TBAB) betrachtet. TBAB stellt eine Leitsubstanz dar, die im Feed in der höchsten Konzentration auftritt und im Kristallisat als Reinigungsfaktor am leichtesten meßbar ist. Die Reaktionsgemische können nach bekannten Verfahren zur Herstellung von Diarylcarbonaten gewonnen werden, beispielsweise gemäß DE-A 19 605 167. In den folgenden Beispielen werden jedoch nur die relevanten Bestandteile DPC, Phenol und TBAB aufgeführt.

**Beispiele**

Reaktionsgemische mit jeweils insgesamt 600 g an DPC, Phenol und TBAB, wie in den folgenden Tabellen angegeben (in Beispiel 1: 106,2 g DPC, 489 g Phenol und 4,8 g TBAB), wurden in einen Rohrbündel-Kristaller als Feed gegeben; durch den getakteten Einsatz mehrerer Kristaller konnte eine quasi-kontinuierliche Fahrweise realisiert werden. Die Temperatur betrug am Anfang 53°C; die Temperatur beim Impfen und damit beim Kristallisationsbeginn ist jeweils angegeben. Nach einer Haltezeit wurde die Restschmelze ("Schmelze" = Mutterlauge Mula) abgelassen. Danach wurde mit dem Abschmelzen des Kristallisats begonnen ("Rein-schmelze"); Heizrate, Heizzeit und Heizendtemperatur hierzu sind jeweils angegeben. Die beim Abschmelzen zuerst anfallenden Teilmengen wurden mit der Mutterlauge (Mula) vereinigt. Die Mengenbilanzen sind angegeben. Die Mutterlauge mit dem gesamten Katalysatorsystem wurde zur Herstellung weiterer Reaktionsgemische eingesetzt. Bei wiederholter Rückführung der Mutterlauge wird ein Anteil von einigen % des Rückführstroms ausgeschleust und ersetzt. Die Reinschmelze wurde destillativ aufgearbeitet.

$$\text{*TBAB Abreicherungsfaktor} = \frac{\text{Masse TBAB Feed / Masse DPC Feed}}{\text{Masse TBAB Rein-Schmelze / Masse DPC Rein-Schmelze}}$$

**Beispiel 1: DPC-Konz. = 17,7 %**

| Kristallisationsbedingungen | | |
|---|---|---|
| Kristallisationstemp. | [°C] | 29 |
| Impftemp. | [°C] | 29 |
| Kühlendtemp. | [°C] | 20 |
| Kühlrate | [K/h] | 2 |
| Kühlzeit | [h] | 4,5 |
| Haltezeit | [h] | 1 |

(fortgesetzt)

| Kristallisationsbedingungen | | |
|---|---|---|
| | | |
| Heizendtemp. | [°C] | 34 |
| Heizrate | [K/h] | 2 |
| Heizzeit | [h] | 7 |

| | Masse [g] | DPC [g] | Phenol [g] | TBAB [g] | Abreicherungs-Faktor TBAB |
|---|---|---|---|---|---|
| Feed | 600 | 106,2 | 489,0 | 4,80 | |
| Schmelze (Mula) | 471,0 | 78,6 | 387,6 | 4,76 | |
| Reinschmelze | 129,0 | 27,6 | 101,4 | 0,04 | 35 |

| Raum-Zeit-Ausbeute | [kg/m3*h] | 3,3 |
|---|---|---|
| DPC-Ausbeute | [%] | 26 |

**Beispiel 2: DPC-Konz. = 48,2 %**

| Kristallisationsbedingungen | | |
|---|---|---|
| Kristallisationstemp. | [°C] | 44 |
| Impftemp. | [°C] | 44 |
| Kühlendtemp. | [°C] | 42,5 |
| Kühlrate | [K/h] | 0,5 |
| Kühlzeit | [h] | 3 |
| Haltezeit | [h] | 1 |
| | | |
| Heizendtemp. | [°C] | 51,5 |
| Heizrate | [K/h] | 2 |
| Heizzeit | [h] | 4,5 |

| | Masse [g] | DPC [g] | Phenol [g] | TBAB [g] | Abreicherungs-Faktor TBAB |
|---|---|---|---|---|---|
| Feed | 600,0 | 289,2 | 307,4 | 3,36 | |
| Schmelze (Mula) | 432,1 | 176,7 | 252,2 | 3,24 | |
| Reinschmelze | 167,9 | 112,5 | 55,3 | 0,12 | 11 |

| Raum-Zeit-Ausbeute | [kg/m3*h] | 22 |
|---|---|---|
| DPC-Ausbeute | [%] | 38,9 |

**Beispiel 3: DPC-Konz. = 69,0 %**

| Kristallisationsbedingungen | | |
|---|---|---|
| Kristallisationstemp. | [°C] | 51,5 |
| Impftemp. | [°C] | 51,5 |
| Kühlendtemp. | [°C] | 48 |
| Kühlrate | [K/h] | 0,5 |
| Kühlzeit | [h] | 7 |
| Haltezeit | [h] | 1 |
| | | |
| Heizendtemp. | [°C] | 74,0 |
| Heizrate | [K/h] | 2 |
| Heizzeit | [h] | 13 |

| | Masse [g] | DPC [g] | Phenol [g] | TBAB [g] | Abreicherungs-Faktor TBAB |
|---|---|---|---|---|---|
| Feed | 600,0 | 414,0 | 182,64 | 3,36 | |
| Schmelze (Mula) | 359,6 | 253,0 | 103,44 | 3,18 | |
| Reinschmelze | 240,4 | 161,0 | 79,2 | 0,18 | 7,4 |

| Raum-Zeit-Ausbeute | [kg/m3*h] | 18 |
|---|---|---|
| DPC-Ausbeute | [%] | 38,9 |

**Patentansprüche**

1. Methode zur Rückgewinnung von Katalysatorsystemen, die einen Platingruppenmetall-Katalysator, einen Cokatalysator, ein quaternäres Salz und eine Base enthalten, aus Reaktionsgemischen zur Herstellung von Diarylcarbonaten der Formel (I)

$$R\text{-}O\text{-}CO\text{-}O\text{-}R \tag{I}$$

durch oxidative Carbonylierung der zugrundeliegenden aromatischen Hydroxyverbindungen der Formel (II)

$$R\text{-}O\text{-}H \tag{II},$$

wobei in den Formeln

R   substituiertes oder nichtsubstituiertes $C_6$-$C_{15}$-Aryl bedeutet,

und die einen Diarylcarbonatgehalt von mindestens 15 und weniger als 70 Gew.-%, bezogen auf das Gesamtgewicht der Reaktionsgemische, aufweist,
dadurch gekennzeichnet, daß man

   a) das Reaktionsgemisch aus dem Reaktor zur Herstellung des Diarylcarbonats in einen zur Schmelzkristallisation geeigneten Apparat überführt,

b) im geeigneten Apparat durch Temperaturabsenkung und Impfen die Schmelzkristallisation einleitet,

c) das Schmelzkristallisat, das überwiegend aus Diarylcarbonat und der zugrundeliegenden aromatischen Hydroxyverbindung besteht, von der zurückbleibenden katalysatorhaltigen Restschmelze abtrennt,

d) das Schmelzkristallisat auf reines Diarylcarbonat und reine aromatische Hydroxyverbindung aufarbeitet und

e) die katalysatorhaltige Restschmelze in den Reaktor zur Herstellung des Diarylcarbonats zurückführt oder zur Gewinnung von Wertstoffen aufarbeitet.

2. Verfahren nach Anspruch 1, dadurch gekennzeichnet, daß der Apparat für die Schmelzkristallisation ein Rohrbündel-Kristallisator oder ein Plattenwärmeaustauscher-Kristallisator ist.

3. Verfahren nach Anspruch 1, dadurch gekennzeichnet, daß das Reaktionsgemisch die Temperatur von einer Starttemperatur im Bereich von 53 bis 29°C mit einer Kühlrate von 20 bis 0,1K/h auf eine Endtemperatur im Bereich von 20 bis 48°C abgesenkt wird.

4. Verfahren nach Anspruch 1, dadurch gekennzeichnet, daß im Schritt b) das Einleiten der Schmelzkristallisation durch Impfen mit festem Diarylcarbonat, fester aromatischer Hydroxyverbindung oder einem Gemisch beider als Impfgut vorgenommen wird.

5. Verfahren nach Anspruch 4, dadurch gekennzeichnet, daß zum Impfen Schmelzkristallisat aus einem vorangegangenen Reaktionslauf eingesetzt wird.

6. Verfahren nach Anspruch 4, dadurch gekennzeichnet, daß 0,02 bis 1 Gew.-% Impfgut, bezogen auf im Reaktionsgemisch vorliegendes Diarylcarbonat, eingesetzt werden.

7. Verfahren nach Anspruch 1, dadurch gekennzeichnet, daß im Schritt c) vor der Abtrennung der Restschmelze eine oder mehrere Haltezeit(en) von 1 bis 5 Stunden eingehalten wird (werden).

8. Verfahren nach Anspruch 1, dadurch gekennzeichnet, daß im Schritt d) das Schmelzkristallisat vor der Aufarbeitung durch "Schwitzen" behandelt wird, wobei die Temperatur mit einer Heizrate von 20 bis 0,1K/h in den Bereich von 29 bis 75°C angehoben wird und weitere entstandene Restschmelze abgetrennt und mit der zuerst entstandenen Restschmelze vereinigt wird.

9. Verfahren nach Anspruch 8, dadurch gekennzeichnet, daß im Verlaufe des "Schwitzens" eine oder mehrere Haltezeit(en) von 1 bis 5 Stunden eingehalten wird (werden).

**Claims**

1. Method for recovering catalyst systems containing a platinum-group-metal catalyst, a co-catalyst, a quaternary salt and a base from reaction mixtures for producing diaryl carbonates of formula (I)

$$R-O-CO-O-R \tag{I}$$

by oxidative carbonylation of the underlying aromatic hydroxy compounds of formula (II)

$$R-O-H \tag{II},$$

wherein in the formulae

R   signifies substituted or non-substituted $C_6$-$C_{15}$-aryl,

and having a diaryl carbonate content of at least 15 and less than 70 wt %, referred to the total weight of the reaction mixtures,

characterised in that

a) the reaction mixture is transferred from the reactor for producing diaryl carbonate into an apparatus suitable for melt crystallization,

b) the melt crystallization is initiated in the suitable apparatus by temperature reduction and inoculation,

c) the melt crystallizate, which consists predominantly of diaryl carbonate and the underlying aromatic hydroxy compound, is separated from the catalyst-containing residual melt that is left,

d) the melt crystallizate is worked up to pure diaryl carbonate and pure aromatic hydroxy compound and

e) the catalyst-containing residual melt is returned into the reactor for producing diaryl carbonate or worked up in order to obtain valuable materials.

2. Method according to claim 1, characterised in that the apparatus for the melt crystallization is a tube bundle crystallizer or a plate heat exchanger crystallizer.

3. Method according to claim 1, characterised in that the reaction mixture temperature is reduced from a starting temperature in the range from 53 to 29 °C with a cooling rate of 20 to 0.1 K/h to a final temperature in the range from 20 to 48 °C.

4. Method according to claim 1, characterised in that in step b) the initiation of the melt crystallization is undertaken by inoculation with solid diaryl carbonate, solid aromatic hydroxy compound or a mixture of both as inoculation material.

5. Method according to claim 4, characterised in that melt crystallizate from a previous reaction run is used for the inoculation.

6. Method according to claim 4, characterised in that 0.02 to .1 wt % of inoculation material, referred to diaryl carbonate present in the reaction mixture, is used.

7. Method according to claim 1, characterised in that in step c) one or more holding time(s) of 1 to 5 hours is (are) observed prior to the separation of the residual melt.

8. Method according to claim 1, characterised in that in step d) the melt crystallizate is treated by "sweating" prior to the working up, wherein the temperature is increased at a heating rate of 20 to 0.1 K/h into the range of 29 to 75 °C and further residual melt obtained is separated and united with the residual melt first obtained.

9. Method according to claim 8, characterised in that one or more holding time(s) of 1 to 5 hours is (are) observed in the course of the "sweating".


**Revendications**

1. Procédé de récupération d'une composition catalytique, qui contient un catalyseur avec un métal du groupe du platine, un cocatalyseur, un sel quaternaire et une base, d'un mélange réactionnel pour la préparation de carbonate de diaryle de formule (I) :

$$R-O-CO-O-R \hspace{6cm} (I)$$

par carbonylation oxydative des composés hydroxylés aromatiques qui en sont la base, de formule (II) :

$$R-O-H \hspace{7cm} (II)$$

où dans les formules :

EP 0 801 052 B1

R représente aryle $C_6$-$C_{15}$ substitué ou non substitué,
et qui présente une teneur en carbonate de diaryle d'au moins 15 et inférieure à 70% en poids, sur base du poids total du mélange de réaction,
qui est caractérisé en ce que l'on :

a) fait passer le mélange de réaction du réacteur pour la préparation du carbonate de diaryle dans un appareil approprié pour la cristallisation de mélange en fusion ;
b) déclenche, dans l'appareil approprié, la cristallisation de mélange fondu par diminution de la température et introduction de germe ;
c) sépare le cristallisat de mélange en fusion, qui consiste essentiellement en carbonate de diaryle et le composé hydroxylé aromatique qui en est la base, de la masse en fusion résiduelle contenant la catalyseur, restant ;
d) traite le cristallisat de fusion en carbonate de diaryle pur et composé hydroxylé aromatique pur, et
e) ramène la masse en fusion résiduelle contenant le catalyseur dans le réacteur de préparation du carbonate de diaryle ou le traite pour récupérer les matières de valeur.

2. Procédé suivant la revendication 1, caractérisé en ce que l'appareil pour la cristallisation de mélange en fusion est un cristallisoir à faisceau tubulaire ou un cristallisoir-échangeur de chaleur à plaques.

3. Procédé suivant la revendication 1, caractérisé en ce que l'on diminue la température du mélange réactionnel depuis une température de départ située dans l'intervalle allant de 53 à 29°C, avec une vitesse de refroidissement allant de 20 à 0,1 K/heure, jusqu'à une température finale située dans l'intervalle allant de 20 à 48°C.

4. Procédé suivant la revendication 1, caractérisé en ce que dans l'étape b), le déclenchement de la cristallisation de mélange en fusion est effectué par introduction de germe avec du carbonate de diaryle solide, le composé hydroxylé aromatique solide ou un mélange des deux comme germe.

5. Procédé suivant la revendication 4, caractérisé en ce que pour l'introduction de germe, on met en oeuvre un cristallisat de fusion d'une réaction précédente.

6. Procédé suivant la revendication 4, caractérisé en ce que l'on met en oeuvre, de 0,02 à 1% en poids de germe, sur base du carbonate de diaryle présent dans le mélange réactionnel.

7. Procédé suivant la revendication 1, caractérisé en ce que dans l'étape c), avant la séparation de la masse en fusion résiduelle, on maintient une ou plusieurs durées de repos de 1 à 5 heures.

8. Procédé suivant la revendication 1, caractérisé en ce que dans l'étape d), le cristallisat de fusion est traité avant le traitement par « exsudation », où la température est augmentée à une vitesse de chauffage allant de 20 à 0,1 K/heure dans l'intervalle allant de 29 à 75°C et séparé de la masse en fusion résiduelle et purifié avec la masse en fusion résiduelle formée d'abord.

9. Procédé suivant la revendication 8, caractérisé en ce que au cours de « l'exsudation », on maintient une ou plusieurs durées de repos allant de 1 à 5 heures.

9